# EUROPEAN PATENT APPLICATION

(11) **EP 4 796 984 A1**
(43) Date of publication of application: **26.08.2026**
(21) Application number: 25305243.5
(22) Date of filing: 24.02.2025
(51) Int. Cl.: G02C 13/00, A61B 3/11, A61B 5/107

(54) **A METHOD FOR MEASURING AT LEAST ONE GEOMETRICO-MORPHOLOGICAL PARAMETER OF A SUBJECT WEARING AN EYEWEAR AND AN ASSOCIATED SYSTEM**

(71) Applicant: Essilor International, 94220 Charenton-Le-Pont (FR)
(72) Inventor: ALLIONE, Pascal, 94130 NOGENT SUR MARNE (FR); GUEGAN, Julien, 94160 SAINT-MANDE (FR); XU, Qi, 77420 CHAMPS SUR MARNE (FR)
(74) Representative: Jacobacci & Partners France

(57) **Abstract**

The invention relates to a method for measuring at least one geometrico-morphological parameter of a subject (2) wearing an eyewear (3) in order to fit an ophthalmic lens in said eyewear. The method comprises the following steps:
- placing the subject wearing the eyewear in a natural posture while looking at a visual symbol (17) among a plurality of visual symbols (9) on a visual target (6),
- placing a double-sided image capture apparatus (5) between the subject and the visual target, with a first image capture device (10) facing the subject, a second image capture device (11) facing the visual target,
- obtaining images of both the subject and visual target thanks to the double-sided image capture apparatus (5),
- determining at least one first point (18) and at least one second point (19) from each of the images,
- identifying the visual symbol,
- determining the at least one geometrico-morphological parameter using the at least one first and second points and the identification of the visual symbol.

The invention also relates to an associated system and a fitting method.

## Description

### TECHNICAL FIELD OF THE INVENTION

The invention relates to a method for determining a value of at least one geometrico-morphological parameter of a subject wearing an eyewear.

This method may be used to determine a vision correction equipment customized for this subject.

### BACKGROUND INFORMATION AND PRIOR ART

Manufacturing an ophthalmic lens adapted to a subject and an eyewear frame in order to provide the subject with appropriate ophthalmic equipment requires the determination of a number of fitting data for fitting the lens in the chosen eyewear.

In order to obtain these fitting data, the determination of geometrico-morphological parameters of the subject is necessary.

Numerous documents describe devices and methods for determining such parameters. These parameters include for example the interpupillary distance of the subject and/or the fitting height, the fitting height corresponding to the vertical distance between the pupil of the eye of the subject and the bottom edge of the eyewear or lens worn by the subject, in conditions of use.

Known methods usually require the intervention of an eye care professional. For example, the eye care professional may manually measure the fitting height with a ruler.

Other known methods comprise using a head mounted device in order to use precise eye trackers to measure gaze direction. However, these methods cannot measure the frame position on the face of the wearer and often require appropriate calibration before use.

Alternatively, existing devices based on the treatment of images of the head of the subject wearing the chosen eyewear can be used. However, these devices work under the condition that the subject looks at a designated point for the measurement.

Therefore, these existing methods usually place the subject in an unnatural posture, wherein a gaze direction of the subject is constrained, and/or does not allow the measurement of the frame position on the face of the subject and/or requires calibrations beforehand. Accuracy of the values of the geometrico-morphological parameter, in particular, the gaze direction(s) and/or the fitting heights are therefore sometimes decreased by the measurement methods.

Moreover, known methods sometimes require the use of specific instruments that are not easily available to the general public.

### SUMMARY OF THE INVENTION

Therefore, one object of the invention is to provide a new method for determining an accurate value of at least one geometrico-morphological parameter of a subject wearing an eyewear, in order to fit an ophthalmic lens in said eyewear.

The above object is achieved according to the invention by providing a method wherein the following steps are performed:
a. placing the subject wearing the eyewear in a natural posture, in which at least one of the gaze directions of the subject points to a visual symbol among a plurality of visual symbols on a visual target,
b. placing a double-sided image capture apparatus between the subject and the visual target, a first image capture device of the double-sided image capture apparatus facing the subject and comprising a first referential, a second image capture device of the double-sided image capture apparatus facing the visual target and comprising a second referential,
c. obtaining at least one first image of an upper body of the subject wearing the eyewear using the first image capture device of the double-sided image capture apparatus, while the subject wearing the eyewear is placed in the natural posture of step a.,
d. obtaining at least one second image of the visual target using the second image capture device of the double-sided image capture apparatus,
e. determining a first position of at least one first point of the first image in the first referential,
f.determining a second position of at least one second point of the second image in the second referential,
g. identifying the visual symbol to which at least one of the gaze directions of the subject pointed at in step a.,
h. determining the at least one geometrico-morphological parameter of the subject wearing the eyewear using the first position in the first referential, the second position in the second referential, a relative positioning of the first image capture device compared to the second image capture device and the identification provided in step g.

Thanks to the method described in the present disclosure, at least one geometrico-morphological parameter of the subject wearing the eyewear, can be determined in a fast and easy way. Advantageously, the subject remains in a natural posture during the measurement, without constraint, which can improve the accuracy of fitting measurements obtained through the at least one geometrico-morphological parameter. Indeed, the proposed method allows a measurement to be made without influencing the subject's gaze direction. The disclosed method is also noninvasive compared for instance to known eye-tracking method and puts no obstacle at a near-field distance from the subject.

Furthermore, the measurement protocol is improved and simplified compared to other known methods wherein preliminary calibrations might be necessary.

The measurement process may be fully automated and does not require the assistance of an eye care professional.

Other advantageous and optional features of the method according to the invention are as follows:
- the first image capture device is a three-dimensional image capture device, and the second image capture device is also a three-dimensional image capture device, the first image of the upper body of the subject wearing the eyewear is a first three-dimensional image, and the second image of the visual target is a second three-dimensional image,
- the at least one first and second three-dimensional images, each comprises a depth matrix and a reflectance image associated to the depth matrix,
- the step of identifying the visual symbol to which at least one of the gaze directions of the subject pointed at in step a., comprises receiving an identification of said visual symbol from the subject,
- the at least one first point comprises a center of the pupil of the subject,
- the at least one first point comprises a corneal light reflection center of gravity,
- the at least one first point comprises a remarkable point of the eyewear worn by the subject,
- the at least one second point comprises a center of gravity of said visual symbols,
- the at least one first point is identified by an image processing algorithm and/or the at least one second point is identified by an image processing algorithm,
- the relative positioning of the first image capture device compared to the second image capture device is used to define a global referential of the double-sided image capture apparatus, and wherein the at least one geometrico-morphological parameter of the subject wearing the eyewear is measured in the global referential,
- the first image capture device and the second image capture device are fixed with respect to each other or comprise means for measuring their relative positionings,
- a size of each visual symbol of the plurality of visual symbols and/or a distance between each visual symbol of the plurality of visual symbols are adapted to the distance between the visual target and the subject,
- the visual target is placed at a distance at least 2 meters aways from the subject, the distance between each of the visual symbols being less than 7 centimeters, each of the visual symbols having a vertical dimension between 1 and 20 centimeters and a horizontal dimension between 1 and 20 centimeters, and the visual target having a vertical dimension of at least 1.4 meter,
- the least one geometrico-morphological parameter of a subject wearing an eyewear comprises at least one of the following:
- a direction of the gaze axis,
- a position of a point of intersection between the lens of the eyewear placed in front of the eye of the subject and the gaze,
- a fitting height,
- an interpupillary distance,
- an eye lens distance.

The invention further relates to a method for fitting an ophthalmic lens in an eyewear worn by a subject, wherein at least one geometrico-morphological parameter of said subject wearing the eyewear obtained thanks to the method for measuring at least one geometrico-morphological parameter as previously described.

The invention also concerns a system for measuring at least one geometrico-morphological parameter of a subject wearing an eyewear in order to determine at least one fitting parameter of an ophthalmic lens in said eyewear according to the method above, the system comprising:
- a double-sided image capture apparatus,
- a visual target comprising a plurality of visual symbols adapted to be placed in front of the subject, such that the subject is placed in a natural posture, and
- a calculating means, configured to receive an input identifying one visual symbol among the plurality of visual symbol of the visual target,

the image capture apparatus is configured to be placed between the subject and the visual target and comprises a first image capture device configured to capture at least one image of an upper body of the subject and a second image capture device configured to capture at least one image of the visual target,
   the first image capture device and the second image capture device being joined together, and
the calculating means is programmed for determining the at least one geometrico-morphological parameter of the subject wearing the frame from the image of the upper body of the subject, the image of the visual target and the input provided.

### DETAILED DESCRIPTION OF EXAMPLE(S)

The following description with reference to the accompanying drawings will make it clear what the invention consists of and how it can be achieved. The invention is not limited to the embodiment illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:
- Figure 1 is a schematic perspective view of a subject and an embodiment of a system according to the invention, the system comprising a double-sided image capture apparatus and a poster.
- Figure 2 is a schematic representation of a first image of the subject and a second image of the poster captured using the double-sided image capture apparatus of figure 1.
- Figure 3 is a block diagram of the steps of an embodiment of a method according to the invention.

In the following, the wording "natural" posture is defined as a posture in which the position of the head 1 of a subject 2 is not imposed by a physical constraint such as applying a part of the head 1 against a surface and/or a posture wherein a subject 2 is looking straight ahead 1 in the distance at eye level in a free manner, i.e. without being given instructions to look at a specific item or symbol. In other words, the subject 2 is considered to be standing or sitting upright and looking straight ahead 1, meaning with his/her gaze in a straight direction. This natural posture is also referred to as the orthostatic posture, wherein the individual achieves minimal efforts.

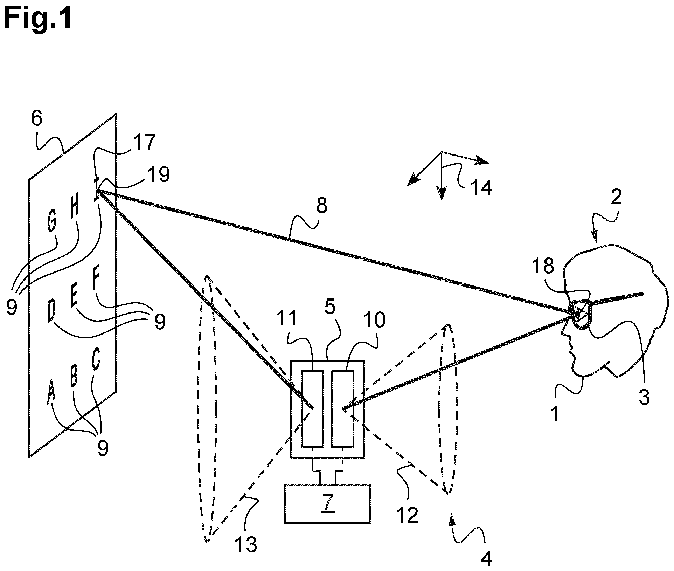

In figure 1, the head 1 of a subject 2, wearing an eyewear 3 or a frame, is represented schematically.

In order to measure at least one geometrico-morphological parameter of the subject 2 wearing the eyewear 3 in order to fit an ophthalmic lens in the eyewear 3 a method according to the invention is implemented by a system 4.

This system 4 is represented in figure 1, and comprises a double-sided image capture apparatus 5, a visual target 6 and a calculating means 7.

By geometrico-morphological parameter, it is referred to, for instance, a parameter linked to both the head 1 of the subject 2 and the chosen eyewear 3, for example, whose value depends on the relative position of the eyewear 3 when fitted on the head 1 of the subject 2. It can also refer to a parameter linked only to the morphology of the subject 2, for instance, whose value depends on the shape of the head 1 of the subject 2 and/or on the position of the features of the head 1 of the subject 2, such as the relative positions of the eyes. Alternatively, it can refer to a parameter linked only to the geometry of the eyewear 3, for example, whose value depends only on the shape of the eyewear 3.

The subject 2, who is wearing the eyewear 3, is supposed to be sitting or standing in the natural posture defined above. In this natural posture, it is supposed that the subject 2 is looking straight ahead 1 at eye-level, without any constraint. Corresponding gaze directions (or directions of gaze axis) in the natural posture are referred to as the primary gaze directions.

As illustrated in figure 1, the visual target 6 is placed in front of the subject 2 while the subject 2 is assuming the natural posture. Therefore, the subject 2 is able to look straight ahead 1 at the visual target 6, while looking at eye-level. For instance, a geometrical center of the visual target 6 is placed at eye-level compared to the subject 2, who can either be sitting or standing. Here the gaze direction is represented by a gaze axis 8 illustrated on figure 1.

The double-sided image capture apparatus 5 is placed between the subject 2 and the visual target 6.

For instance, in a preferred embodiment, the visual target 6 is a poster, placed on a wall in front of the subject 2 or placed vertically on a mount.

Alternatively, the visual target 6 can correspond to a screen placed in front of the subject 2, which is configured to display visual information dynamically, for instance, a computer screen.

Here, the visual target 6 is placed at a certain distance from the subject 2. For instance, if the geometrico-morphological parameter is to be determined in condition of far vision, the visual target 6 should be placed as far as possible from the subject 2. This would ensure a measurement in a natural posture, while the subject 2 is comfortably looking straight ahead 1.

For instance, the visual target 6 can be placed two meters away from the subject 2. Furthermore, here, the visual target 6 has a height of 1.4 meters and a width of 1.4 meters. The height of the visual target 6 is measured along a vertical axis when the visual target 6 is placed in front of the subject 2, while the width of the visual target 6 is measured along a horizontal axis in similar conditions. These axes are measured relative to the subject 2, while the system 4 is placed in a working orientation.

In alternative embodiments, the visual target 6 has various values of height and/or width. In particular, the height and/or width of the visual target 6 can be chosen to be smaller or bigger than 1.4 meters. Indeed, the height of the visual target 6 and the width of the visual target 6 are determined according to an extent of gaze directions covered for a given distance between the subject 2 and the visual target 6, while said subject 2 is facing a geometrical center of the visual target 6.

The visual target 6, e.g. the poster in the first embodiment, comprises a plurality of visual symbols 9. For instance, as represented in figure 1, the plurality of visual symbols 9 can refer to a series of letters. In variants, the plurality of visual symbols 9 might refer to numbers, or geometrical shapes, or photos, or pictures or an assembly of letters and/or numbers and/or geometrical shapes and/or photos.

Preferably, visual symbols 9 of the plurality of visual symbols 9 on the visual target 6 all have the same overall size i.e. a same overall height and overall width in the plane of the visual target 6. This overall size is also referred to as apparent size of the visual symbol 9. However, the visual symbols 9 might present different colors.

Advantageously, all visual symbols 9 are different from one another, either by shape or/and by colors. Therefore, the visual symbols 9 are easily identified and named by the subject 2.

The visual symbols 9 of the visual target 6 are arranged regularly, or irregularly onto a surface of the visual target 6. However, a number and a spacing between the visual symbols 9 are configured to cover a large interval of height and/or width onto the visual target 6.

For instance, the number and the spacing of the visual symbols 9 are defined according to the distance between the subject 2 and the visual target 6 plane and according to the height and width of the visual target 6. In particular, the number and the spacing of the visual symbols 9 is advantageously chosen to cover the extent of gaze directions between minus 15 degrees and plus 15degrees along both horizontal and vertical axes. The spacing (i.e. the distance between two adjacent visual symbols 9) should be large enough so each visual symbol 9 is easily distinguishable from the adjacent visual symbols 9, while remaining small enough to ensure a natural posture, wherein the subject 2 can look in a forward direction and see at least one visual symbol 17. Said otherwise, the subject 2 does not need to deviate his/her gaze direction from the primary gaze direction to be able to see a visual symbol 17 on the visual target 6. In the exemplary embodiment currently described, wherein the visual target 6 is two meters away from the subject 2, a distance between two adjacent visual symbols 9 should be under 7 centimeters. This distance between two adjacent visual symbols 9 is for instance measured along the vertical axis and/or the horizontal axis.

The overall size of the visual symbols 9 can also be adjusted, for instance based on the distance between two adjacent visual symbols 9. Alternatively, the overall size of the visual symbols 9 and/or the distance between two adjacent visual symbols 9 is chosen according to an accuracy of the double-sided image capture apparatus 5 and a precision requirement on the geometrico-morphological parameter which is intended to be measured.

For instance, if the geometrico-morphological parameter to be measured corresponds to a fitting height, and if the precision requirement is equal for instance to 1 millimeter, an overall height of the visual symbol 9 should be at least 7 centimeters. Indeed, a projection of a viewed angle onto the surface of the visual target 6, which is here equal to the overall height of the visual symbol 9 is defined according to the precision requirement, the given distance between the subject 2 and the visual target 6 and a distance between the eye rotation center and a front side of the lens of the eyewear.

Here, each visual symbol 9 has an overall width of between 1 and 20 centimeters, for instance 7 centimeters and an overall height between 1 and 20 centimeters, for instance 7 centimeters.

The visual symbols 9 and the visual target 6 are thus arranged to cover a large field of view for the subject 2. Therefore, a given visual target 6 is able to accommodate a large array of different subjects 2 with different heigh, different visual field, different ages, etc. This ensures that different subjects 2 placed at a certain distance from a given visual target 6 might all be able to look at the visual symbols 9 in a natural posture, i.e. with their head 1 straight while looking in the forward direction.

However, in another possible embodiment, the visual target 6 can be adapted to a distance between the visual target 6 and the subject 2. For instance, if the visual target 6 comprises a screen which can dynamically be configured to display visual information, the apparent size of the visual symbols 9 can be adapted to the distance between the visual target 6 and the subject 2. Therefore, the apparent size of the visual symbols 9 remains the same regardless of that distance.

As previously indicated, the double-sided image capture apparatus 5 is positioned between the subject 2 and the visual target 6. For instance, a position of the double-sided image capture apparatus 5 can be aligned compared to an axis drawn between the head 1 or head 1 and trunk of the subject 2 and the geometrical center of the visual target 6. In this case, the double-sided image capture apparatus 5, the subject 2 and the geometrical center of the visual target 6 appears to be aligned. However, the position of the double-sided image capture apparatus 5 can be arbitrary compared to the subject 2 and the geometrical center of the visual target 6. In particular, a distance between the subject 2 and the double-sided image capture apparatus 5 is arbitrary.

The double-sided image capture apparatus 5 comprises a first image capture device 10 and a second image capture device 11, placed back-to-back compared to each other.

The first image capture device 10 and the second image capture device 11 can either correspond to a same model, with identical properties or to different models of image capture devices with different properties.

The first image capture device 10 is configured to capture an image of the subject 2, for instance his/her head 1 or his/her head 1 and his/her trunk, i.e. an upper body of the subject 2. This image is referred to as a first image.

The first image capture device 10 comprises an imaging optical system. The first image capture device 10 is positioned so that the imaging optical system is facing the subject 2. In particular, the first image capture device 10 has a first field of view 12 represented on figure 1, as a cone, which is defined by the imaging optical system.

The position of the first image capture device 10 is so that the subject 2, in particular, the head 1 of the subject 2 is at least partially included in the first field of view 12 in order to be imaged by the first image capture device 10.

The second image capture device 11 is configured to capture at least one image of the visual target 6. Therefore, the second image capture device 11 faces the visual target 6. In particular, the second image capture device 11 comprises an imaging optical system which defines a second field of view 13 for the second image capture device 11. The visual target 6 is at least partially included in the second field of view 13 in order to be imaged by the second image capture device 11.

Hence, as long as the first image capture device 10 is able to capture an image of the subject 2, and the second image capture device 11 is able to capture an image of the visual target 6, the double-sided image capture apparatus 5 can be placed at an arbitrary position with an arbitrary orientation between the subject 2 and the visual target 6.

In the preferred embodiment, the first and second image capture devices 10, 11 are placed back-to-back compared to each other, in order to form the double-sided image capture apparatus 5. For instance, the first and second image capture devices 10,11 are fixed with respect to each other, with rigid mechanical means.

Alternatively, they might comprise means for measuring a relative positioning between them.

In the embodiment represented in figure 1, it is considered that the first image capture device 10 and second image capture device 11 are fixed with respect to each other. Thus, they may not be displaced with respect to each other.

A relative positioning of the first image capture device 10 compared to the second image capture device 11 is used to define a global referential 14 of the double-sided image capture apparatus 5.

In particular, the first image capture device 10 comprises a first referential, which comprises three orthogonal axes and an origin. This first referential can be used to describe three-dimensional coordinates of all elements surrounding the first image capture device 10.

Likewise, the second image capture device 11 comprises a second referential, which also comprises three orthogonal axis and an origin. All elements surrounding the second image capture device 11 also have three-dimensional coordinates in the second referential.

As the first referential and second referential do not share an origin and/or the orthogonal axes may or may not have the same orientation, a given element possesses different three-dimensional coordinates in the first compared to the second referential.

As in the preferred embodiment, the first image capture device 10 and the second image capture device 11 are fixed with respect to each other, it is possible to define a translation matrix and a rotation matrix. These matrixes define the relative positioning of the first and second referential, i.e. a relative positioning of the first image capture device 10 compared to the second image capture device 11. It is therefore possible to define the global referential 14, associated with the double-sided image capture apparatus 5. For instance, the first referential is used as the global referential 14. Three-dimensional coordinates defined in the second referential can be converted into the global referential 14 (i.e. the first referential) using the rotational matrix and the translation matrix.

This global referential 14 is shown in figure 1. It comprises three orthogonal axis and an origin

All elements surrounding the double-sided image capture apparatus 5 have therefore three-dimensional coordinates in this global referential 14 which allow their localization in this global referential 14.

In order to retrieve the three-dimensional coordinates in the global referential 14, the double-sided image capture apparatus 5 comprises means for acquiring three-dimensional information. In particular, the three-dimensional coordinates are first retrieved in the first referential and in the second referential.

For instance, in the first embodiment, the first image capture device 10 comprises a first three-dimensional image capture device and the second image capture device 11 comprises a second three-dimensional image capture device. The first three-dimensional image capture device and the second three-dimensional image capture device are configured to capture images in opposite directions.

The first three-dimensional image capture device is configured to face the subject 2 and to capture a three-dimensional image of the subject 2, while the second three-dimensional image capture device is configured to face the visual target 6 and to capture a three-dimensional image of the visual target 6. These three-dimensional images captured by the double-sided image capture apparatus 5 are used to retrieve three-dimensional information of both the visual target 6 and the subject 2, in particular, three-dimensional coordinates of points of interest.

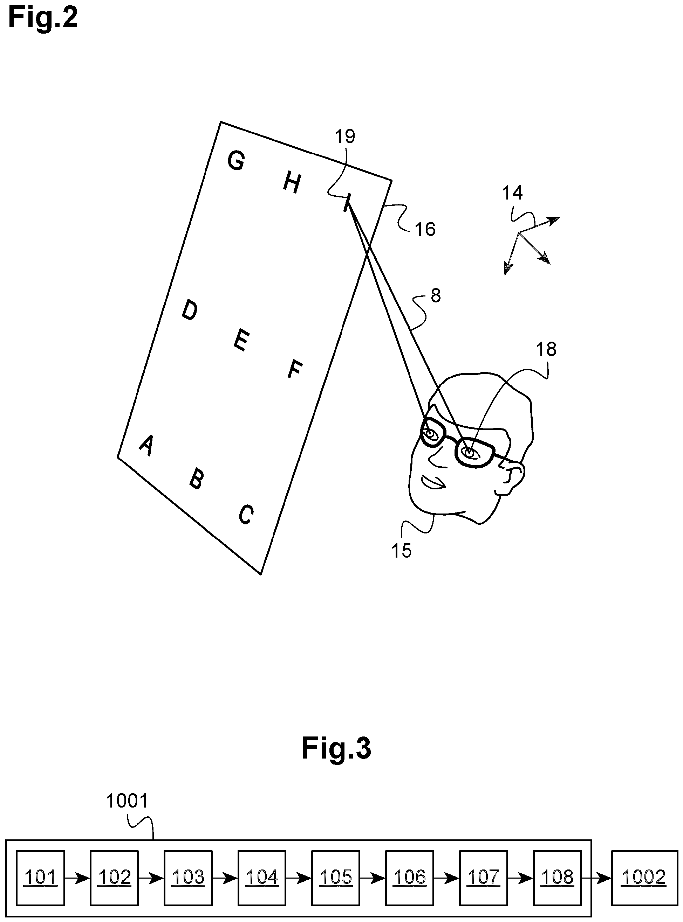

Examples of a first image 15 captured by the first image capture device and of a second image 16 captured by the second image capture device 10 are shown on figure 2.

Here, the three-dimensional image capture devices are configured to acquire three-dimensional images, each of them comprising a color image, a depth matrix and a two-dimensional map linking the color image to the depth matrix.

To acquire the color image, also referred to as a reflectance image, which is two-dimensional, the first and second three-dimensional image capture devices 10, 11comprise an array of pixels. The pixels from this array of pixels are each identified by their position in a row and in a column, therefore, each pixel is identified by its coordinates (i,j).

In the first embodiment, the color image corresponds to a RGB color image, hence, pixels of the array of pixels are divided into subpixels.

Each subpixel is photosensitive to a specific color, i.e. red, green, blue, and measure an intensity associated with each color. In particular, in a given pixel, the three subpixels are arranged from left to right as red, green and blue.

In other words, the three-dimensional image capture device comprises three sub-arrays of subpixels, which are each sensitive to a specific color. These sub-arrays are configured to acquire matrixes of sub-pixels for each color.

The matrix of sub-pixels comprises a matrix associated with red M_{red}(i,j), a matrix associated with green M_{green}(i,j) and a pixel associated with blue M_{blue}(i,j). Hence, a pixel of the three-dimensional image is associated with a value from each of the matrixes if sub-pixels which form the color image.

Pixels of the array of pixels, identified by the their coordinates (i,j) are also associated with a value from the depth matrix z(i,j). This depth matrix z(i,j) provides for pixels of the array of pixels a depth information.

This depth matrix z(i,j) is acquired for instance with a stereovision measuring means, or a time-of-flight measuring means, comprised in the three-dimension image capture device. The stereovision measuring means, or stereo vision camera, acquires images from at least two different vantage points, in order to extract three-dimensional information of a given scene.

Alternatively, the time-of-flight measuring means preferably uses an infrared light source and image sensor. Such a time-of-flight measuring means provides a depth information by measuring a time-of-flight between the time-of-flight measuring means and a reflective object, which backscatters light emitted by time-of-flight measuring means.

In particular, for the first image capture device 10, the depth information is measured between the three-dimensional image capture device and the subject 2 while for the second image capture device 11, the depth information is measured between the three-dimensional image capture device and the visual target 6.

Finally, the three-dimensional image capture device is configured to record a two-dimensional map [x(i,j), y(i,j)], which links each pixel (i,j) of the color image to a three-dimensional point, described by a triplet of coordinate [x(i,j), y(i,j), z(i,j)] of coordinates in a referential. In other words, the two-dimensional map [x(i,j), y(i,j)] corresponds to the positions of the pixels recorded by the three-dimensional image capture device. Hence, each sub-pixel of the matrixes of sub-pixels M_{red}(i,j), M_{green}(i,j), M_{blue}(i,j) that forms the reflectance image is associated to a three-dimensional point described by three-dimensional coordinates in the referential.

In another possible embodiment, the three-dimensional image capture device records only a depth matrix and an infrared reflectance image instead of a RGB color reflectance image. Alternatively, the reflectance image can be in greyscale.

In the first embodiment, the first image 15 of the subject 2 acquired by the first three-dimensional image capture device comprises triplets of coordinates for points of the subject 2 wearing the eyewear 3 in the first referential. Likewise, the second image 16 of the visual target 6 acquired by the second three-dimensional image capture device comprises triplets of coordinates for points of the visual target 6 in the second referential. Hence, as previously explained, the coordinates corresponding to the visual target 6 can be converted to the first, i.e., the global referential 14.

The system 4 also comprises a calculating means 7. This calculating means 7 is functionally connected to the first image capture device 10 and the second image capture device 11 and is configured to receive the first image 15 and the second image 16 once captured.

For instance, the calculating means 7 is comprised in a computer.

This calculating means 7 is programmed for determining at least one geometrico-morphological parameter of the subject 2 wearing the eyewear 3, based on the first image 15 and the second image 16 captured respectively by the first image capture device 10 and the second image capture device 11.

In particular, the calculating means 7 is also configured to receive an input identifying one visual symbol 9 among the plurality of visual symbols 9 present on the visual target 6. To this end, the calculating means 7 is functionally connected to a microphone and/or a keyboard, and/or a touch screen, and/or an image capture device for instance.

The calculating means 7, in an exemplary embodiment, is further programmed to determine the at least one geometrico-morphological parameter of the subject 2 wearing the frame taking into account the input received.

The system 4 described above is therefore configured to implement a method for measuring at least one geometrico-morphological parameter of the subject 2 wearing an eyewear 3, in order to fit an ophthalmic lens in the eyewear 3. In the first embodiment, the method comprises steps, which are illustrated in the flowchart of figure 3.

A first step 101 comprises placing the subject 2 wearing the eyewear 3 in a natural posture, in which at least one of the gaze directions of the subject 2 points to a visual symbol 17 among a plurality of visual symbols 9 on a visual target 6. In other words, the subject 2 wearing the eyewear 3 is looking at one of the visual symbols 9 of the visual target 6 while in the natural posture and his/her gaze direction remaining in the primary gaze direction.

This step 101 is made possible thanks to the previously described system 4, which comprises an advantageously configured visual target 6. In particular, placing the visual target 6 far away enough from the subject 2 allows said subject 2 to keep a natural posture, with his/her gaze direction in the primary gaze direction. The spacing and size of the visual symbols 9 is configured so that the subject 2 can naturally gaze at a visual symbol 9 while remaining in the natural posture.

Optionally, the natural posture can be verified by an eye care professional, or through an assessment of landmarks of the head 1 and/or the trunk of the subject 2, as detailed below.

Then, the method involves a step 102 of placing the double-sided image capture apparatus 5 described above between the subject 2 and the visual target 6. Therefore, the first image 15 capture device 9 of the double-sided image capture apparatus 5, which is associated with the first referential, faces the subject 2. The second image capture device 11, which is here fixed at the back of the first image capture device 10, faces the visual target 6. The second image capture device 11 is associated with the second referential. Given that the relative position of the first and second image capture device 10,11 is known, a global referential 14 can be defined, as detailed in the description of the system 4 according to the invention.

In practice, the double-sided image capture apparatus 5 and the visual target 6 can remain at a given position, and the subject 2 would be positioned accordingly, i.e., facing the visual target 6, with the double-side image capture apparatus in-between.

The next step 103 is to obtain at least one first image 15 of an upper body (head and trunk) of the subject 2 wearing the eyewear 3 using the first image capture device 10 of the double-sided image capture apparatus 5. The at least one first image 15 is obtained while the subject 2 wearing the eyewear 3 is placed in the natural posture exposed above. For instance, the subject 2 can be told to remain still for a few seconds for the first image capture device 10 to record the first image 15.

Here, according to the first embodiment of the system 4 described above, wherein the double-sided image capture apparatus 5 comprises two three-dimensional images capture devices, corresponding to the first image capture device 10 and to the second image capture device 11, the first image 15 is a three-dimensional image. In particular, the first image 15 comprises both a first depth matrix and a first color image linked together through a first two-dimensional map. Points corresponding to features of the subject 2 wearing the eyewear 3 are therefore acquired by pixels of the first image capture device 10. Each pixel is associated with three color values, one from each of the matrix of sub-pixels M_{red}(i,j), M_{green}(i,j), M_{blue}(i,j) of the color image and with a triplet of three-dimensional coordinates [x(i,j), y(i,j), z(i,j)] in the first referential. Said otherwise, points corresponding to features of the subject 2 wearing the eyewear 3 are located with coordinates in the first referential, the points being also associated with a color information from the color image.

For instance, the first image 15 comprises at least part of the head 1 and/or trunk of the subject 2. In particular, the first image 15 comprises in the first embodiment of the method, as represented in figure 2, the face of the subject 2 and the eyewear 3 he/she is wearing.

The method also comprises a step 104 of obtaining at least one second image 16 of the visual target 6 using the second image capture device 11 of the double-sided image capture apparatus 5. An example of the second image 16 is represented in figure 2.

For instance, obtaining the second image 16 of the visual target 6 using the second image capture device 11 corresponds to a step of acquiring an image.

This step can either occur simultaneously before or after compared to the step of obtaining the first image 15 of the subject 2 wearing the eyewear 3.

In another embodiment of the method, obtaining the second image 16 of the visual target 6 using the second image capture device 11 can refer to accessing a memory unit of the second image capture device 11 to retrieve a second image 16 captured in a previous acquisition step. This embodiment is possible if the double-sided image capture apparatus 5 has remained still relative to the visual target 6 since the aforementioned previous acquisition step.

Advantageously however, the double-sided image capture apparatus 5 can freely be moved before and/or between acquiring the first image 15 and the second image 16, without requiring a calibration. In the first case, wherein the double-sided image capture apparatus 5 has been moved before obtaining the first and the second images, both first and second images are captured again. In the second case, wherein the double-sided image capture apparatus 5 has been moved after acquiring the first, respectively the second image 16, a new second, respectively first image 15 has to be captured by the corresponding image capture device.

Hence, the position of the double-sided image capture apparatus 5 can easily be changed, without requiring any calibration step, neither before nor during the implementation of the method.

The method then requires a step 105 of determining a first position of at least one first point 17 of the first image 15 in the first referential. This step 105 can either occur after the first image 15 has been obtained, or once both the first and second images have been obtained.

This step 105 of determining a first position of at least one first point 18 allows to retrieve three-dimensional coordinates of at least a landmark feature of the head 1 and/or trunk of the subject 2 and/or the eyewear 3 he/she is wearing. A position of a single landmark feature or positions of a set of landmark features can be retrieve in the first referential. In other words, based on the first image 15, remarkable points of the physical space, described by their coordinates in the first referential, are determined.

For instance, the determination of the first position of the at least one first point 18 is based on the first color image (or reflectance image) on which image processing algorithms are applied. A correspondence between the coordinates (i,j) of pixels representative of the landmark feature(s) of interest and their position in the physical space is then established with the first depth matrix and the first two-dimensional map to determine the first position. This first position is described by a triplet of coordinates in the first referential.

This step is implemented by the calculating means 7, or by a dedicated image processing unit.

The image processing algorithms applied comprise for instance a shape detection algorithm, like a circle detection algorithm, or a machine learning algorithm suitably trained beforehand to detect certain landmark features in a color image.

The at least one first point 18 refers to several possible landmark features of the head 1 and/or trunk of the subject 2 and/or the eyewear 3 he/she is wearing, for instance, it can refer to a center of the pupil of the subject 2, a corneal light reflection center of gravity, a remarkable point of the eyewear 3 worn by the subject 2. In some embodiment, it can refer to a set of landmark features, and would therefore comprise several first points 18.

Here, in the currently described first embodiment of the method, a first position, defined by a triplet of coordinates in the first referential is retrieved by an image processing algorithm. The first position would localize the center of the pupil of one of the eyes of the subject 2.

The method also involves a step 106 of determining a second position of at least one second point 19 of the second image 16 in the second referential. Similarly to the previously described step of determining the first position, this step 106 can for instance occur after obtaining the second image 16.

This step 106 retrieves at least one second point 19 corresponding to a landmark feature on the visual target 6, in particular, it retrieves its position in the second referential.

This step 106 is for instance implemented thanks to the image processing algorithms previously mentioned which are applied to the second color image of the second image 16. Relevant pixels from the second color image are detected by the image processing algorithms (either pattern detection algorithm, or properly trained machine learning algorithm). These relevant pixels are then matched to their second position in the second referential, thanks to the second depth matrix and the second two-dimensional map of the second image 16.

This step 106 is for instance implemented by the calculating means 7, or by a dedicated image processing unit.

The at least one second point 19 refers to several possible landmark features of the visual target 6. For example, it refers to a center of gravity of the visual symbols 9 of the visual target 6.

For instance, the step 106 for determining a second position of at least one second point 19 of the second image 16 in the second referential corresponds to determining second positions for second points 18 associated with each visual symbol 9 of the visual target 6.

Here, in the first embodiment of the method, this step 106 provides several second positions, each defined by a triplet of coordinates in the second referential. The several positions comprise a center of gravity for each of the visual symbols 9 represented on the visual target 6. For instance, according to the second image 16 of the visual target 6 represented on figure 2, each visual symbol 9 i.e., each letter from "A" to "I" is associated with a second positions.

A subsequent step 107 comprises identifying the visual symbol 17 to which the at least one of the gaze directions of said subject 2 pointed at in the step 101 of placing the subject 2 wearing the eyewear 3 in a natural posture. In other words, the visual symbol 17 at which the subject 2 looked at when starring in a straight direction toward the visual target 6 while placed in the natural posture, is identified.

The identification is for instance done by the subject 2 himself/herself, who communicates at which visual symbol 17 he/she looked at previously. For instance, he/she can inform the eye care professional supervising the method which transforms this information into an input identifying one visual symbol 17 among the plurality of visual symbols 9. This input identifying one visual symbol 17 among the plurality of visual symbols 9 is for instance received by the calculating means 7 through a microphone and/or a keyboard, and/or a touch screen for instance. Alternatively, this task can be performed by the subject 2 himself/herself.

Here, for instance, it is considered that the identification of the visual symbol 9 the subject 2 gazed at is provided by the subject 2, through a keyboard.

Once the first position of the at least one first point 18 and the second position of the at least one second point 19 has been established, and once the visual symbol 17 has been identified it is proceeded with a step 108 of determining the at least one geometrico-morphological parameter of the subject 2 wearing the eyewear 3. This step 108 is implemented by using the first position in the first referential, the second position in the second referential, the relative positioning of the first image capture device 10 compared to the second image capture device 11 and the identification of the visual symbol 9

In particular, the first position and the second position are converted into the global referential 14. This is achieved through the rotation matrix and translation matrix, i.e. the solid kinematics which describe the relative positioning of the first image capture device 10 compared to the second image capture device 11. Here, given the system 4 described, the rotation matrix and the translation matrix are used to convert the second position in the second referential to a second position in the first referential, i.e., the global referential 14 associated with the double-sided image capture apparatus 5.

Based on the first position and the second position, all possible geometrico-morphological parameters based on these positions are determined. At least one geometrico-morphological parameter is then selected among all the possible geometrico-morphological parameters based on the identification of the visual symbol 17.

For instance, both the coordinates of the center of the pupil of the subject 2 and the coordinates of the center of gravity of each visual symbol 9 comprised in the visual target 6 (the letters from "A" to "I"), which are determined in the second referential, based on the second image 16, are then converted to the first referential.

Based on the coordinates of the center of the pupil and the center of gravity of each visual symbol 9 a set of possible gaze directions for one of the eyes of the subject 2 can be determined. In particular, for each of the visual symbol 9 the coordinates of the center of the pupil and the coordinates of a center of gravity of one of the visual symbols 9, are used to determine a possible gaze direction. For instance, the possible gaze directions are determined by calculating a three-dimensional vector between the coordinates of the center of the pupil and the coordinates of the center of each of the visual symbols 9. Each three-dimensional vector defines a possible three-dimensional gaze direction, for one of the eyes of the subject 2, which can be represented by three angles in the global referential 14. However, in practice, only two angles are usually necessary, for instance, when determining a fitting height or determining an interpupillary distance, as only two angles are relevant in the plane of the ophthalmic lens of the eyewear 3.

Then, based on the identification of the visual symbol 17 gazed at by the subject 2, one gaze direction 19 is selected among the set of possible gaze directions.

Optionally, the selected gaze direction 19 is then used to calculate a fitting height for one of the eyes of the subject 2 wearing an eyewear 3. To do so, a lens plane of the eyewear 3 worn by the subject 2 is determined, for instance, using the first image 15 captured by the first image capture device 10. Then, an intersection between the selected gaze direction and the lens plane is used to calculate a fitting height.

Therefore, in this example, the method allows the measurement of two geometrico-morphological parameters of the subject 2 wearing an eyewear 3, the gaze direction and the associated fitting height.

In possible embodiments, the least one geometrico-morphological parameter of a subject 2 wearing an eyewear 3 comprises at least one of the following: a direction of the gaze axis, which is commonly referred to as the gaze direction, a position of a point of intersection between the lens of the eyewear 3 placed in front of the eye of the subject 2 and the gaze, a fitting height, an interpupillary distance, an eye lens distance (i.e. a distance between the eye of the subject 2, and the lens plane).

The obtained at least one geometrico-morphological parameter can be used in a method for fitting an ophthalmic lens in an eyewear 3 worn by a subject 2. This method is illustrated on figure 3. One step 1001 of this fitting method comprises implementing the measurement method to measure at least one geometrico-morphological parameter of the subject 2 wearing the eyewear 3. The geometrico-morphological parameter obtained is used in a subsequent step 1002 for fitting the ophthalmic lens in the eyewear 3. For instance, in a possible embodiment, the fitting method comprises determining the gaze direction and fitting height of each eye of the subject 2 and using the fitting heights to design and produce a suitable ophthalmic lens.

## Claims

1. Method for measuring at least one geometrico-morphological parameter of a subject (2) wearing an eyewear (3) in order to fit an ophthalmic lens in said eyewear (3), wherein the following steps are performed:
a. placing the subject (2) wearing the eyewear (3) in a natural posture, in which at least one of the gaze directions of the subject (2) points to a visual symbol (17) among a plurality of visual symbols (9) on a visual target (6),
b. placing a double-sided image capture apparatus (5) between the subject (2) and the visual target (6), a first image capture device (10) of the double-sided image capture apparatus (5) facing the subject (2) and comprising a first referential, a second image capture device (11) of the double-sided image capture apparatus (5) facing the visual target (6) and comprising a second referential,
c. obtaining at least one first image (15) of an upper body of the subject (2) wearing the eyewear (3) using the first image capture device (10) of the double-sided image capture apparatus (5), while the subject (2) wearing the eyewear (3) is placed in the natural posture of step a.,
d. obtaining at least one second image (16) of the visual target (6) using the second image capture device (11) of the double-sided image capture apparatus (5),
e. determining a first position of at least one first point (18) of the first image (15) in the first referential,
f. determining a second position of at least one second point (19) of the second image (16) in the second referential,
g. identifying the visual symbol (17) to which at least one of the gaze directions of said subject (2) pointed at in step a.,
h. determining the at least one geometrico-morphological parameter of the subject (2) wearing the eyewear (3) using the first position in the first referential, the second position in the second referential, a relative positioning of the first image capture device (10) compared to the second image capture device (11) and the identification provided in step g.

2. The method according to claim 1, wherein the first image capture device (10) is a three-dimensional image capture device, and the second image capture device (11) is also a three-dimensional image capture device, the first image (15) of the upper body of the subject (2) wearing the eyewear (3) is a first three-dimensional image, and the second image (16) of the visual target (6) is a second three-dimensional image.

3. The method according to any one of claims 1 or 2, wherein the at least one first and second three-dimensional images each comprise a depth matrix and a reflectance image associated to the depth matrix.

4. The method according to any one of claims 1 to 3, wherein the step of identifying the visual symbol (17) to which at least one of the gaze directions of the subject (2) pointed at in step a., comprises receiving an identification of said visual symbol (17) from the subject (2).

5. The method according to any one of claims 1 to 4, wherein the at least one first point (18) comprises a center of the pupil of the subject (2).

6. The method according to any one of claims 1 to 5, wherein the at least one first point (18) comprises a corneal light reflection center of gravity.

7. The method according to any one of claims 1 to 6, wherein the at least one first point (18) comprises a remarkable point of the eyewear (3) worn by the subject (2).

8. The method according to any one of claims 1 to 7, wherein the at least one second point (19) comprises a center of gravity of said visual symbols (9).

9. The method according to any one of claims 1 to 8, wherein the at least one first point (18) is identified by an image processing algorithm and/or the at least one second point (19) is identified by an image processing algorithm.

10. The method according to any one of claims 1 to 9, wherein the relative positioning of the first image capture device (10) compared to the second image capture device (11) is used to define a global referential (14) of the double-sided image capture apparatus (5), and wherein the at least one geometrico-morphological parameter of the subject (2) wearing the eyewear (3) is measured in the global referential (14).

11. The method according to any one of claims 1 to 10, wherein the first image capture device (10) and the second image capture device (11) are fixed with respect to each other or comprise means for measuring their relative positionings.

12. The method according to any one of claims 1 to 11, wherein a size of each visual symbol (9) of the plurality of visual symbols (9) and/or a distance between each visual symbol (9) of the plurality of visual symbols (9) are adapted to the distance between the visual target (6) and the subject (2).

13. The method according to claim 12, wherein the visual target (6) is placed at a distance at least 2 meters aways from the subject (2), the distance between each of the visual symbols (9) being less than 7 centimeters, each of the visual symbols (9) having a vertical dimension between 1 and 20 centimeters and an horizontal dimension between 1 and 20 centimeters, and the visual target (6) having a vertical dimension of at least 1.4 meter.

14. The method according to any one of claims 1 to 13, wherein the least one geometrico-morphological parameter of a subject (2) wearing an eyewear (3) comprises at least one of the following:
- a direction of the gaze axis,
- a position of a point of intersection between the lens of the eyewear (3) placed in front of the eye of the subject (2) and the gaze,
- a fitting height,
- an interpupillary distance,
- an eye lens distance.

15. Method for fitting an ophthalmic lens in an eyewear (3) worn by a subject (2), wherein at least one geometrico-morphological parameter of said subject (2) wearing the eyewear (3) obtained thanks to the method for measuring at least one geometrico-morphological parameter according to any one of claims 1 to 14 is used for fitting the ophthalmic lens in the eyewear (3).

16. System (4) for measuring at least one geometrico-morphological parameter of a subject (2) wearing an eyewear (3) in order to determine at least one fitting parameter of an ophthalmic lens in said eyewear (3) according to the method described in any one of claims 1 to 15, comprising:
- a double-sided image capture apparatus (5),
- a visual target (6) comprising a plurality of visual symbols (9) adapted be placed in front of the subject (2), such that the subject (2) is placed in a natural posture, and
- a calculating means (7), configured to receive an input identifying one visual symbol (17) among the plurality of visual symbol (9) of the visual target (6),
the double-sided image capture apparatus (5) is configured to be placed between the subject (2) and the visual target (6) and comprises a first image capture device (10) configured to capture at least one first image (15) of an upper body of the subject (2) and a second image capture device (11) configured to capture at least one second image (16) of the visual target (6),
the first image capture device and the second image capture device being joined together, and
the calculating means (7) is configured for determining the at least one geometrico-morphological parameter of the subject (2) wearing the eyewear (3) from the first image (15) of upper body of the subject (2), the second image (16) of the visual target (6) and the input provided.
